# EUROPEAN PATENT SPECIFICATION

(11) **EP 3 132 819 B1**
(45) Date of publication and mention of the grant of the patent: **10.04.2019**
(21) Application number: 15780404.8
(22) Date of filing: 27.02.2015
(51) Int. Cl.: G16H 20/17, A61M 5/142, A61M 5/172

(54) **INTELLIGENTLY-ANALGESIC INFUSION PUMP MONITORING SYSTEM AND METHOD**
ÜBERWACHUNGSSYSTEM UND VERFAHREN FÜR INTELLIGENTE ANALGETISCHE INFUSIONSPUMPE
SYSTÈME ET PROCÉDÉ DE SURVEILLANCE DE POMPE À PERFUSION D'ANALGÉSIQUE INTELLIGENT

(30) Priority: 18.04.2014 CN 201410156960
(43) Date of publication of application: 22.02.2017
(73) Proprietor: Yu, Weizhong, Chengdu, Sichuan 610072 (CN)
(72) Inventor: YU, Weizhong, Chengdu Sichuan 610072 (CN); YU, Wenkai, Chengdu Sichuan 610072 (CN)
(74) Representative: Isarpatent
(86) International application number: PCT/CN2015/073385
(87) International publication number: WO 2015/158184

(56) References cited:
- WO-A2-02/11049
- WO-A2-2005/057466
- CN-A- 1 966 100
- CN-A- 101 732 778
- CN-A- 102 592 063
- CN-A- 103 623 482
- CN-A- 103 920 208
- CN-U- 201 519 339
- CN-U- 203 861 681
- CN-U- 203 861 684
- US-A1- 2011 004 188
- LU PING ET AL: "A Data Acquisition System for Methane Drainage Based on ARM and FPGA", COMPUTER DISTRIBUTED CONTROL AND INTELLIGENT ENVIRONMENTAL MONITORING (CDCIEM), 2011 INTERNATIONAL CONFERENCE ON, IEEE, 19 February 2011 (2011-02-19), pages 1-3, XP031938451, DOI: 10.1109/CDCIEM.2011.451 ISBN: 978-1-61284-278-3
- LIFENG XIAO ET AL: "Design and realization of strain measurement system based on FPGA and ARM", INTELLIGENT CONTROL AND INFORMATION PROCESSING (ICICIP), 2013 FOURTH INTERNATIONAL CONFERENCE ON, IEEE, 9 June 2013 (2013-06-09), pages 853-857, XP032441851, DOI: 10.1109/ICICIP.2013.6568191 ISBN: 978-1-4673-6248-1
- BRITTON B ET AL: "DESIGN AN FPGA-BASED PCI BUS INTERFACE", ELECTRONIC DESIGN, PENTON MEDIA, CLEVELAND, OH, US, vol. 43, no. 5, 6 March 1995 (1995-03-06), pages 100-105, XP000511403, ISSN: 0013-4872

## Description

### Technical Field

The present invention relates to a medical appliance, and more particularly, to a system and method for monitoring an infusion pump capable of intelligently easing pain.

### Background Art

An electronic infusion pump plays an increasing important role in relieving the sharp pain of patients after operation and other pain-suffered patient, and improving the life quality; and thus has been widely applied in clinic. Due to individual differences of patients, problems appeared during the application of the infusion pump are relatively apparent, for example: individualized drug application according to the demands of the patients cannot be realized sometimes due to lacking of effective tutelage and real time monitoring, so that risks caused by insufficient or excessive medication cannoot be avoided. Insufficient medication will cause incomplete analgesia, while excessive medication will cause respiratory depression, blood pressure drop, all-over pruritus, uroschesis, lethargy and the like, which will even cause threat to life in case of being not found and treated in time. In order to monitor the infusion dose of the patient, a manner of ward rounds manually conducted by doctors is presently employed, which although is straggled no matter on timeliness or availability.

Intelligent infusion pumps are developed earlier at abraod, for example, those countries like Japan, USA and Germany have developed infusion pumps since late 1980s. Prevalent infusion pumps on the market presently are most foreign products, which have various types and better performances, for example, 0T-601 infusion pump (the control accuracy is 10%) and SP-500 syringe pump of Japan JMS, GeminiPC-2TX infusion pump of US MED Corporation which can realize four-way control, Multifuse, Perfusor Compact (the control accuracy may reach 2%), Infusomat P and Infusomat fmS of Germany B.BRAUN (B.BRAUN). There are numerous types of infusion pumps, and Isreal also has corresponding products. Infusion pummp has been developed in middle 1980s at home, which starts relatively late. Although there are some domestic infusion pumps on the market, for example, ZNB series infusion pump products of Shenzhen Kangfute Corporation, and Beijing Keilifenggao Science and Technology Development Co., Ltd., the type of the domestic infusion pumps is fewer and the performances thereof need to be further improved generally. The infusion pumps will be developed towards an orientation of being more portable, smaller, and being more accurate, safer and more reliable to control.

The following documents relate to further technological background of the present invention:

| | |
|---|---|
| D1 | WO 02/11049 A2 (SIMS DELTEC INC [US]) 7 February 2002 (2002-02-07) |
| | |
| D2 | WO 2005/057466 A2 (CARDINAL HEALTH 303 INC [US]) 23 June 2005 (2005-06-23) |
| | |
| D3 | LU PING ET AL: "A Data Acquisition System for Methane Drainage Based on ARM and FPGA", |
| | COMPUTER DISTRIBUTED CONTROL AND INTELLIGENT ENVIRONMENTAL MONITORING (CDCIEM), 2011 INTERNATIONAL CONFERENCE ON, IEEE, 19 February 2011 (2011-02-19), pages 1-3, XP031938451, |
| | DOI: 10.1109/CDCIEM.2011.451 |
| | ISBN: 978-1-61284-278-3 |
| | |
| D4 | LIFENG XIAO ET AL: "Design and realization of strain measurement system based on FPGA and ARM", |
| | INTELLIGENT CONTROL AND INFORMATION PROCESSING (ICICIP), 2013 FOURTH INTERNATIONAL CONFERENCE ON, IEEE, 9 June |
| | |
| | 2013 (2013-06-09), pages 853-857, XP032441851, |
| | DOI: 10.1109/ICICIP.2013.6568191 |
| | ISBN: 978-1-4673-6248-1 |

Document D1 discloses a system for monitoring an infusion pump capable of intelligently easing pain, comprising at least a terminal device unit and a monitoring server, a network server, a computer and a medical pump. There is sensing of conditions by means of sensors and the infusion pump is used for delivering a pain relief medication by means of an application. The terminal device unit comprises at least an infusion pump control terminal and the infusion pump control terminal is connected with the monitoring server. Document D1 also discloses that the control system comprises an external communication sensor which can sense data from an attached remote gathering device e.g an EKG monitor. The infusion pump comprises also a microprocesssor interconnected through a bus to sensors, a communication port and other peripheral devices.
Document D2 relates to a system and method for network monitoring of multiple medical devices and in particular discloses connecting an infusin pump directly to a server by applying a WIFI protocol and using wireless access points. Documents D3 and D4 disclose applying a FGPA for interconnecting a processor with sensor and other peripherals. D3 and D4 also disclose the application of an ARM processor in a sensor data collection context.

### Summary of the Invention

The object of the present invention aims at overcoming the defects of the prior art, and providing a system and method for monitoring an infusion pump capable of intelligently easing pain, which supports such functions like intellectual infusion control, online monitoring of basic vital sign data of a suffer, online warning of patient online perception and feedback, and the like. This object is solved by a monitoring system of claim 1 and a monitoring method of claim 6. Further advantageous embodiments and improvements of the present invention are listed in the dependent claims. Hereinafter, before coming to a detailed discussion of the embodiments of the invention with reference to the attached drawings, some aspects of the invention which contribute to the understanding of the invention are discussed separately below.

The present invention comprises, in one aspect: a system for monitoring an infusion pump capable of intelligently easing pain includes at least a terminal device unit and a monitoring server, wherein each terminal device unit includes at least a infusion pump control terminal and a wireless AP, and each infusion pump control terminal is connected with the monitoring server through a wireless AP and a local area network respectively.
The infusion pump control terminal at least includes a human body vital sign sensor, an infusion control device, a field programmable gate array FPGA, an ARM processor and a WIFI communication module, wherein each human body vital sign sensor is connected with the signal input end of the field programmable gate array FPGA through a sensor interface circuit respectively, the infusion control device is connected with the control signal output end of the field programmable gate array FPGA, the field programmable gate array FPGA is in communication with the ARM processor in a bus coding mode, and the ARM processor is in communication connection with the wireless AP through the WIFI communication module.
The monitoring server includes an infusion scheme input module for providing an infusion scheme input interface for a doctor, and the ARM processor includes an infusion scheme processing module for converting an infusion scheme into an infusion control device control signal.

The human body vital sign sensor includes any one or combination of a blood pressure sensor, a finger temperature sensor, a pulse sensor, a body temperature sensor and a blood oxygen sensor.

The sensor interface circuit includes an I2C, SPI or RS232 interface circuit.

The infusion pump control terminal further includes an LCD display screen and a warning circuit, wherein the LCD display screen is connected with the ARM processor, and the warning circuit is connected with the field programmable gate array FPGA.

The infusion pump control terminal further includes an input keyboard, wherein the input keyboard is connected with the field programmable gate array FPGA, and the field programmable gate array FPGA includes an online perception feedback information input module for providing an online perception feedback information input interface for a sufferer.

The infusion pump control terminal further includes an infusion flow rate sensor and a liquid medicine level sensor, wherein the infusion flow rate sensor and the liquid medicine level sensor are connected with the signal input end of the field programmable gate array FPGA respectively.

The present invention according to the above aspects has the advantageous effects that:
(1) a plurality of basic vital sign data of the sufferer such as blood pressure, finger temperature, pulse, body temperature and blood oxygen is collected in real time, corresponding infusion schemes are generated through analysis of the data, the infusion pump is controlled to achieve automatic infusion, monitoring and pain-easing infusion are combined together for coordinative work, and infusion control is more scientific and reliable;
(2) remotely centralized monitoring, management and dispatching of a plurality of infusion pumps may be realized, so that the efficiency and the system reliability are improved;
(3) the online perception and feedback of the sufferer are supported, and the system can be helped for self-improvement, so as to obtain a more accurate and reliable infusion scheme; and
(4) infusion flow rate monitoring and liquid level monitoring are supported, infusion abnormality and infusion completion may be judged through the flow rate and liquid level data, and automatic warning can be performed in case of infusion abnormality or infusion completion, to prompt medical care personnel or sufferer to treat in time, thus improving the infusion safety.

### Brief Description of the Drawings

Fig. 1 is a network structure topological graph of a monitoring system according to the present invention;
Fig. 2 is a structure block diagram of an infusion pump control terminal according to the present invention.

### Detailed Description of the Preferred Embodiments

The present invention will be further described hereinafter by reference to the embodiments, but the protection scope of the present invention is not limited to the following descriptions.

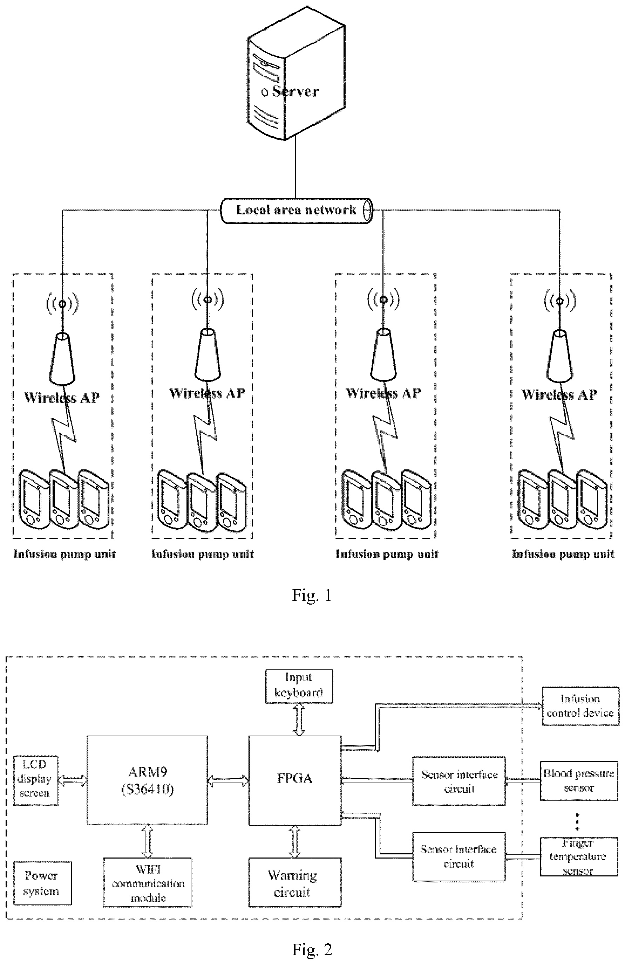

As shown in Fig. 1, a system for monitoring an infusion pump capable of intelligently easing pain includes at least a terminal device unit and a monitoring server, wherein each terminal device unit includes at least a infusion pump control terminal and a wireless AP, and each infusion pump control terminal is connected with the monitoring server through a wireless AP and a local area network respectively.

One or more wireless APs may be disposed in each ward. The wireless AP has two application manners wherein the first manner is to set the wireless AP into a bridging mode, convert a wired network to which the wireless AP is accessed into a wireless network, and a wireless network gateway and an IP address field are kept unchanged, and the quantity of terminals which can be connected with each wireless AP is determined by the quantity of residual IP addresses of the original local area network when using this application manner. The second manner is to set the wireless AP into a routing mode, the wireless APs make up a signle wireless local area network, wherein the network is a subnetwork of the wireless AP accessed to the local area network; and the wireless AP is responsible for routing two network addresses. The quantity of the terminals which can be connected with each wireless AP is more than 200 when using this application manner.

As shown in Fig. 2, the infusion pump control terminal at least includes a human body vital sign sensor, an infusion control device, a field programmable gate array FPGA, an ARM processor and a WIFI communication module, wherein each human body vital sign sensor is connected with the signal input end of the field programmable gate array FPGA through a sensor interface circuit respectively, the infusion control device is connected with the control signal output end of the field programmable gate array FPGA, the field programmable gate array FPGA is in communication with the ARM processor in a bus coding mode, and the ARM processor is in communication connection with the wireless AP through the WIFI communication module. The monitoring server includes an infusion scheme input module for providing an infusion scheme input interface for a doctor, and the ARM processor includes an infusion scheme processing module for converting an infusion scheme into an infusion control device control signal.

An ARM9 S36410 processor is employed as the ARM processor, wherein the processor supports such embedded operating systems as Windows CE, Linux and the like, which facilitates software system development. Meanwhile, the processor has low power consumption and abundant interfaces, supports WIFI communication, LAN communication, USB communication and the like, and is very suitable for developing portable mobile instruments and apparatuses. A system clock of the ARM 9S36410 can reach as high as 800MHz, which can satisfy the requirements on real time control and information interaction.

FPGA is employed for controlling between the ARM processor and the sensor, the infusion control device, and the warning circuit, wherein the abundant I/O resources of the FPGA facilitates system upgrading.

An infusion pump driving module of the infusion control device consists of a two-phase four-wire stepping motor and a pump body. Because accurate and reliable metering are required for infusion, the stepping motor is selected as a power plant. The stepping motor is an actuator which converts changes of pulsed excitation into exact rotor position increment motion, i.e., converts a pulse signal into corresponding angular displacement of a motor. Therefore, the running time of the motor can be controlled through the quantity of control pulses, and the running speed of the motor can be controlled through changing a pulse frequency. The running of the motor drives a cam mechanism of the pump body to compress a rubber tube in a circulating manner to realize the infusion function. In order to make the infusion pump work safely and reliably, and start from the angle of simplifying a hardware circuit, the running of the stepping motor is not directly controlled by a Darlington tube driven by a singlechip; instead, a driving module of the stepping motor is indirectly controlled through two control wires, thus realizing start and control respectively. M008335 is selected as a driving chip of the stepping motor herein. A starting signal starts the stepping motor to work, while the running time and stopping time are controlled by a control signal, and the infusion speed is controlled through the difference of the stopping and rotating time of the stepping motor.

The human body vital sign sensor includes any one or combination of a blood pressure sensor, a finger temperature sensor, a pulse sensor, a body temperature sensor and a blood oxygen sensor.

The sensor interface circuit includes an I2C, SPI or RS232 interface circuit.

The infusion pump control terminal further includes an LCD display screen and a warning circuit, wherein the LCD display screen is connected with the ARM processor, and the warning circuit is connected with the field programmable gate array FPGA.

The infusion pump control terminal further includes an input keyboard, wherein the input keyboard is connected with the field programmable gate array FPGA, and the field programmable gate array FPGA includes an online perception feedback information input module for providing an online perception feedback information input interface for a sufferer. The online perception and feedback information includes: temporarily increased dose (start once in every 24h, and one drop for each time), analgesia score (VAS), motor score (Bromage), ramsay score (Ramsay), patient sense and the like.

The infusion pump control terminal further includes an infusion flow rate sensor and a liquid medicine level sensor, wherein the infusion flow rate sensor and the liquid medicine level sensor are connected with the signal input end of the field programmable gate array FPGA respectively;

Furthermore, the system for monitoring an infusion pump capable of intelligently easing pain further includes the following functions: (1) input and registration of basic data of the patient as well as operation management based on a wireless network; (2) determining such parameters as infusion frequency (drop number/second), total infusion quantity and total liquid medicine quantity and the like according to the individual conditions of the patient through a basic data analysis system; (3) being capable of changing the originally set parameters in any time and temporarily suspending infusion during running; (4) different users may have a set of self-own operating parameters, and respectively record and dynamically monitor the operating parameters; (5) displaying the vital characters of the sufferer through LCD, and being capable of observing the liquid medicine used and the residual content of the liquid medicine, and reporting to a data center instantly; (6) important operations are both completed by two combination keys to prevent misoperation, so that it is safe and reliable; (7) the user makes requests to a monitoring center instantly, and the monitoring center remotely increases or decreases the medicine dose for the user according to the actual situation, and the sufferer may also press the key at a time for increasing the medicine dose (avaliable for one time in every 24h); and (8) all the data can be remotely monitored and recorded online; meanwhile, the system has transfinite control and warning functions.

## Claims

1. A system for monitoring an infusion pump capable of intelligently easing pain, comprising at least a terminal device unit and a monitoring server, wherein each terminal device unit comprises at least a infusion pump control terminal and a wireless AP, and each infusion pump control terminal is connected with the monitoring server through a wireless AP and a local area network respectively, wherein:
a) the infusion pump control terminal at least comprises a human body vital sign sensor, an infusion control device, a field programmable gate array FPGA, an ARM processor and a WIFI communication module, wherein each human body vital sign sensor is connected with the signal input end of the field programmable gate array FPGA through a sensor interface circuit respectively, the infusion control device is connected with the control signal output end of the field programmable gate array FPGA, the field programmable gate array FPGA is in communication with the ARM processor in a bus coding mode, and the ARM processor is in communication connection with the wireless AP through the WIFI communication module; and
b) the monitoring server comprises an infusion scheme input module for providing an infusion scheme input interface for a doctor, and the ARM processor comprises an infusion scheme processing module for converting an infusion scheme into an infusion control device control signal; wherein
c) the infusion pump control terminal further comprises an LCD display screen and a warning circuit, wherein the LCD display screen is connected with the ARM processor, and the warning circuit is connected with the field programmable gate array FPGA.

2. The system for monitoring an infusion pump capable of intelligently easing pain according to claim 1, wherein the human body vital sign sensor comprises any one or combination of a blood pressure sensor, a finger temperature sensor, a pulse sensor, a body temperature sensor and a blood oxygen sensor.

3. The system for monitoring an infusion pump capable of intelligently easing pain according to claim 1, wherein the sensor interface circuit comprises an I2C, SPI or RS232 interface circuit.

4. The system for monitoring an infusion pump capable of intelligently easing pain according to claim 1, wherein the infusion pump control terminal further comprises an input keyboard, wherein the input keyboard is connected with the field programmable gate array FPGA, and the field programmable gate array FPGA comprises an online perception feedback information input module for providing an online perception feedback information input interface for a sufferer.

5. The system for monitoring an infusion pump capable of intelligently easing pain according to claim 1, wherein the infusion pump control terminal further comprises an infusion flow rate sensor and a liquid medicine level sensor, wherein the infusion flow rate sensor and the liquid medicine level sensor are connected with the signal input end of the field programmable gate array FPGA respectively.

## Patentansprüche

1. System zur Überwachung einer Infusionspumpe, die in der Lage ist, auf intelligente Weise Schmerz zu lindern, die mindestens eine Endgerät-Einheit und einen Überwachungsserver umfasst, wobei jede Endgerät-Einheit mindestens einen Infusionspumpen-Steuerungsanschluss und einen Drahtlos-AP umfasst, und jeder Infusionspumpen-Steuerungsanschluss mit dem Überwachungsserver jeweils durch einen Drahtlos-AP und ein Nahbereichsnetz verbunden ist, wobei:
a) der Infusionspumpen-Steuerungsanschluss mindestens einen Patienten-Lebenszeichensensor, eine Infusionsteuerungsvorrichtung, ein feldprogrammierbares Gate-Array (FPGA), einen ARM-Prozessor und ein WIFI-Kommunikationsmodul umfasst, wobei jeder Patienten-Lebenszeichensensor jeweils mit dem Signaleingangsende des feldprogrammierbaren Gate-Array (FPGA) durch eine Sensorschnittstellenschaltung verbunden ist, die Infusionsteuerungsvorrichtung mit dem Steuerungssignalausgangsende des feldprogrammierbaren Gate-Array (FPGA) verbunden ist, das feldprogrammierbare Gate-Array (FPGA) mit dem ARM-Prozessor in einem Bus-Codiermodus kommuniziert und der ARM-Prozessor mit dem Drahtlos-AP durch das WIFI-Kommunikationsmodul in Kommunikationsverbindung steht; und
b) der Überwachungsserver ein Infusionsregime-Eingabemodul zum Bereitstellen einer Infusionsregime-Eingabeschnittstelle für einen Arzt umfasst und der ARM-Prozessor ein Infusionsregime-Verarbeitungsmodul zum Umwandeln eines Infusionsregimes in ein Infusionsteuerungsvorrichtungs-Steuerungssignal umfasst; wobei
c) der Infusionspumpen-Steuerungsanschluss des Weiteren einen LCD-Anzeigebildschirm und eine Warnschaltung umfasst, wobei der LCD-Anzeigebildschirm mit dem ARM-Prozessor verbunden ist und die Warnschaltung mit dem feldprogrammierbaren Gate-Array (FPGA) verbunden ist.

2. System zur Überwachung einer Infusionspumpe, die in der Lage ist, auf intelligente Weise Schmerz zu lindern, nach Anspruch 1, wobei der Patienten-Lebenszeichensensor eines von, oder eine Kombination von, Folgendem umfasst: ein Blutdrucksensor, ein Fingertemperatursensor, ein Pulssensor, ein Körpertemperatursensor und ein Blutsauerstoffsensor.

3. System zur Überwachung einer Infusionspumpe, die in der Lage ist, auf intelligente Weise Schmerz zu lindern, nach Anspruch 1, wobei die Sensorschnittstellenschaltung eine I2C-, SPI- oder RS232-Schnittstellenschaltung umfasst.

4. System zur Überwachung einer Infusionspumpe, die in der Lage ist, auf intelligente Weise Schmerz zu lindern, nach Anspruch 1, wobei der Infusionspumpen-Steuerungsanschluss des Weiteren eine Eingabetastatur umfasst, wobei die Eingabetastatur mit dem feldprogrammierbaren Gate-Array (FPGA) verbunden ist und das feldprogrammierbare Gate-Array (FPGA) ein Online-Wahrnehmungsrückmeldungsinformations-Eingabemodul zum Bereitstellen einer Online-Wahrnehmungsrückmeldungsinformations-Eingabeschnittstelle für einen Patienten umfasst.

5. System zur Überwachung einer Infusionspumpe, die in der Lage ist, auf intelligente Weise Schmerz zu lindern, nach Anspruch 1, wobei der Infusionspumpen-Steuerungsanschluss des Weiteren einen Infusionsdurchflussratensensor und einen Flüssigmedizin-Pegelsensor umfasst, wobei der Infusionsdurchflussratensensor und der Flüssigmedizin-Pegelsensor jeweils mit dem Signaleingangsende des feldprogrammierbaren Gate-Array (FPGA) verbunden sind.

## Revendications

1. Système de surveillance d'une pompe à perfusion apte à apaiser intelligemment la douleur, comprenant au moins une unité formant dispositif terminal et un serveur de surveillance, dans lequel chaque unité formant dispositif terminal comprend au moins un terminal de commande de pompe à perfusion et un point d'accès (AP, *access point*) sans fil, et chaque terminal de commande de pompe à perfusion est connecté au serveur de surveillance par le biais d'un point d'accès sans fil et d'un réseau local, respectivement, dans lequel :
a) le terminal de commande de pompe à perfusion comprend au moins un capteur de signes vitaux du corps humain, un dispositif de commande de perfusion, un circuit intégré prédiffusé programmable FPGA (*field programmable gate array*), un processeur ARM et un module de communication Wi-Fi, dans lequel chaque capteur de signes vitaux du corps humain est connecté à l'extrémité d'entrée de signaux du circuit intégré prédiffusé programmable FPGA par le biais d'un circuit d'interface de capteur, respectivement, le dispositif de commande de perfusion est connecté à l'extrémité de sortie de signaux de commande du circuit intégré prédiffusé programmable FPGA, le circuit intégré prédiffusé programmable FPGA est en communication avec le processeur ARM dans un mode de codage de bus, et le processeur ARM est en liaison de communication avec le point d'accès sans fil par le biais du module de communication Wi-Fi ; et
b) le serveur de surveillance comprend un module d'entrée de schéma de perfusion procurant une interface d'entrée de schéma de perfusion à un médecin, et le processeur ARM comprend un module de traitement de schéma de perfusion destiné à convertir un schéma de perfusion en un signal de commande de dispositif de commande de perfusion ; dans lequel :
c) le terminal de commande de pompe à perfusion comprend en outre un écran d'affichage à cristaux liquides et un circuit d'avertissement, dans lequel l'écran d'affichage à cristaux liquides est connecté au processeur ARM, et le circuit d'avertissement est connecté au circuit intégré prédiffusé programmable FPGA.

2. Système de surveillance d'une pompe à perfusion apte à apaiser intelligemment la douleur, selon la revendication 1, dans lequel le capteur de signes vitaux du corps humain comprend un capteur quelconque ou une combinaison quelconque de capteurs parmi un capteur de pression artérielle, un capteur de température digitale, un capteur de rythme cardiaque, un capteur de température corporelle et un capteur d'oxygène sanguin.

3. Système de surveillance d'une pompe à perfusion apte à apaiser intelligemment la douleur, selon la revendication 1, dans lequel le circuit d'interface de capteur comprend un circuit d'interface I2C, SPI ou RS-232.

4. Système de surveillance d'une pompe à perfusion apte à apaiser intelligemment la douleur, selon la revendication 1, dans lequel le terminal de commande de pompe à perfusion comprend en outre un clavier de saisie, dans lequel le clavier de saisie est connecté au circuit intégré prédiffusé programmable FPGA, et le circuit intégré prédiffusé programmable FPGA comprend un module d'entrée de retour d'information de perception en ligne permettant de procurer une interface d'entrée de retour d'information de perception en ligne à un patient.

5. Système de surveillance d'une pompe à perfusion apte à apaiser intelligemment la douleur, selon la revendication 1, dans lequel le terminal de commande de pompe à perfusion comprend en outre un capteur de débit de perfusion et un capteur de niveau de médicament liquide, dans lequel le capteur de débit de perfusion et le capteur de niveau de médicament liquide sont connectés à l'extrémité d'entrée de signaux du circuit intégré prédiffusé programmable FPGA, respectivement.
